# EUROPEAN PATENT APPLICATION

(11) **EP 1 065 215 A1**
(43) Date of publication of application: **03.01.2001**
(21) Application number: 99112459.5
(22) Date of filing: 30.06.1999
(51) Int. Cl.: C07K 14/705, C12N 15/12, C12N 5/06, G01N 33/566, A61K 38/17

(54) **Homomeric and heteromeric AMPA receptors**

(71) Applicant: Boehringer Ingelheim International GmbH, 55218 Ingelheim am Rhein (DE)
(72) Inventor: Barsoumian, Edward Leon, 560-0005 Osaka Prefecture (JP); IIzuka, Masaki, Hyogo, 666-0129 (JP); Nishimura, Seiichiro, Hyogo, 666-0122 (JP); Akiba, Isamu, Hyogo, 666-0251 (JP)
(74) Representative: Laudien, Dieter, Dr.

(57) **Abstract**

The present invention provides cell lines expressing a recombinant heteromeric AMPA glutamate receptor, preferably a human heteromeric AMPA glutamate receptor, or a recombinant human homomeric AMPA glutamate receptor. The invention also provides methods for preparing said cell lines. The invention furthermore pertains to the use of said cell lines for the identification of glutamate receptor agonists, antagonists or modulators and methods for the identification of said agonists, antagonists or modulators. The invention also provides pharmaceutical compositions comprising said agonists, antagonists or modulators. The invention furthermore is concerned with the use of agonists, antagonists or modulators in the manufacture of a medicament for the treatment of neurological disorders.

## Description

### Technical Field of the Invention

The present invention belongs to the field of neurological research and in particular ionic channels and AMPA receptors.

### Background Art

Glutamate is the major excitatory neurotransmitter in the central nervous system, and its receptor has been classified into four subtypes; the N-methyl-D-aspartate (NMDA) receptors, the α-amino-3-hydroxy-5-methyl-4-isoxazolepropionate (AMPA)/kainate receptors, kainate receptors and the metabotropic receptors (Hollmann and Heinemann, 1994; Jonas and Burnashev, 1995). The glutamate receptors play an important role in memory and learning. On the other hand, excessive activation of glutamate receptors contributes to neuronal injury or cell death "excitotoxicity" in a variety of pathologic conditions, including epilepsy, stroke and various neurodegenerative disorders (Coyle and Puttfarcken, 1993: Pellegrini-Giampietro et al., 1997). It has been reported that the mechanisms underlying the glutamate-induced neurodegeneration involve the excessive influx of Ca²⁺ into neurons through NMDA-type receptors, triggered by the activation of AMPA-type receptors (Yoneda and Ogita, 1991).

Four AMPA receptor subunits (GluR1, 2, 3 and 4) have been cloned, and the functional properties of the receptor clones were elucidated by transient expression studies using *Xenopus* oocytes and mammalian cells (Keinänen et al., 1990; Sommer et al., 1990; Hollmann et al., 1991; Verdoorn et al., 1991; Mosbacher et al., 1994). Each AMPA receptor subunit exists in two different forms, designated "flip" and "flop", created by alternative splicing of a 115-base pair region preceding the transmembrane domain IV. The current amplitudes are 4- to 5-fold larger for glutamate in flip receptors compared to flop forms. The homomeric receptors assembled from GluR1, GluR3, or GluR4 subunit are highly permeable to Ca²⁺, and have doubly rectifying current-voltage (I-V) relationships. In contrast, the receptors assembled from GluR2 subunit alone show a low Ca²⁺ permeability and possess linear or outwardly rectifying I-V relationships.

In heteromeric combinations, co-expression of GluR2 and the other AMPA receptor subunits exhibits the formation of recombinant channels possessing similar profiles obtained with GluR2 subunit alone. The low permeability to Ca²⁺ is attributed to the replacement of a glutamine by an arginine, which occurs by RNA editing, in the pore-lining region of GluR2 subunit (Hume et al., 1991; Burnashev et al., 1992a). The positively charged arginine also results in a loss of sensitivity to intracellular polyamines which cause inward rectification (Kamboj et al., 1995; Washburn et al, 1997).

The native AMPA receptors are thought to be assembled from each of the subunits alone and from their combinations, resulting in the heteromultimeric composition of AMPA receptors with functionally different properties (Burnashev et al., 1992b; Geiger et al., 1995). Therefore, understanding the functional and molecular determinants of AMPA receptors either alone or in combination allows to deduce the molecular characteristics of the native AMPA receptor function.

As mentioned *supra,* the transient expression of the AMPA receptor subunits GluR1, GluR2, GluR3 and GluR4 in *Xenopus* oocytes and mammalian cells is known in the art (Keinänen et al., 1990; Sommer et al., 1990; Hollmann et al., 1991; Verdoorn et al., 1991; Mosbacher et al., 1994). Cells transiently expressing AMPA receptors soon downregulate their receptor expression. Thus, the artisan has to generate new transiently transfected cells before carrying out experiments. Furthermore, there is a large variability in expression from one transient expression to another. Therefore, said transiently transfected cells cannot be used for assays wherein a large number of compounds is tested simultaneously such as high throughput screening assays (HTS), e.g. for the screening for receptor agonists, antagonists or modulaturs (e.g. allosteric modulators) as there is a need for a cell line expressing said receptor at a constant level.

In the art, there are a few documents disclosing attempts to establish stable mammalian cell transfectants expressing the homomeric AMPA receptors using cDNAs derived from different non-human species (Andersen et al., 1996; Hennegriff et al., 1997). The disadvantage of the before-mentioned art is that said transfectants cannot be used to investigate the function of human AMPA receptors or human neurological disorders associated with said receptors. So far, only two stable transfectants expressing the flip splice variants of human homomeric GluR3 (Varney et al., 1998) and the GluR4 AMPA receptors (Fletcher et al., 1995), respectively, have been reported.

It is assumed that native GluR channels in hippocampal neurons are heterooligomers rather than a mosaic of homooligomers (Jonas, 1993, Jonas and Burnashev, 1995). However, primary neuronal cells, as they are post-mitotic cells and have to be freshly prepared for every experiment, are not very useful in many research applications and in particular not in industrial applications such as HTS. Thus, there is an urgent need in the art for cell lines stably expressing a heteromeric AMPA receptor, preferably a human heteromeric AMPA receptor, or a human homomeric AMPA receptor. In particular, there is a requirement in the art for cell lines expressing said receptors wherein said receptors have similar or identical functional characteristics (i.e. as GluR channels) as those expressed in primary neuronal cells.

Therefore, the problem underlying the present invention is to provide cell lines stably expressing a heteromeric AMPA receptor, preferably a human heteromeric AMPA receptor, or a human homomeric AMPA receptor, a process for the identification of an agonist, antagonist or modulator of said receptors, a pharmaceutical composition comprising said agonist, antagonist or modulator and a use for said agonists, antagonists or modulators in the treatment of neurological disorders.

### Summary of the invention

The above-captioned technical problem is solved by the embodiments characterized in the claims and the description. The before-mentioned disadvantages in the art are overcome by the claims and the description of the present invention.

The present invention provides cell lines expressing a recombinant heteromeric AMPA glutamate receptor, preferably a human heteromeric AMPA glutamate receptor, or a recombinant human homomeric AMPA glutamate receptor. The invention also provides methods for preparing said cell lines. The invention furthermore pertains to the use of said cell lines for the identification of glutamate receptor agonists, antagonists or modulators and methods for the identification of said agonists, antagonists or modulators. The invention also provides pharmaceutical compositions comprising said agonists, antagonists or modulators. The invention furthermore is concerned with the use of agonists, antagonists or modulators in the manufacture of a medicament for the treatment of neurological disorders.

### Brief description of the figures

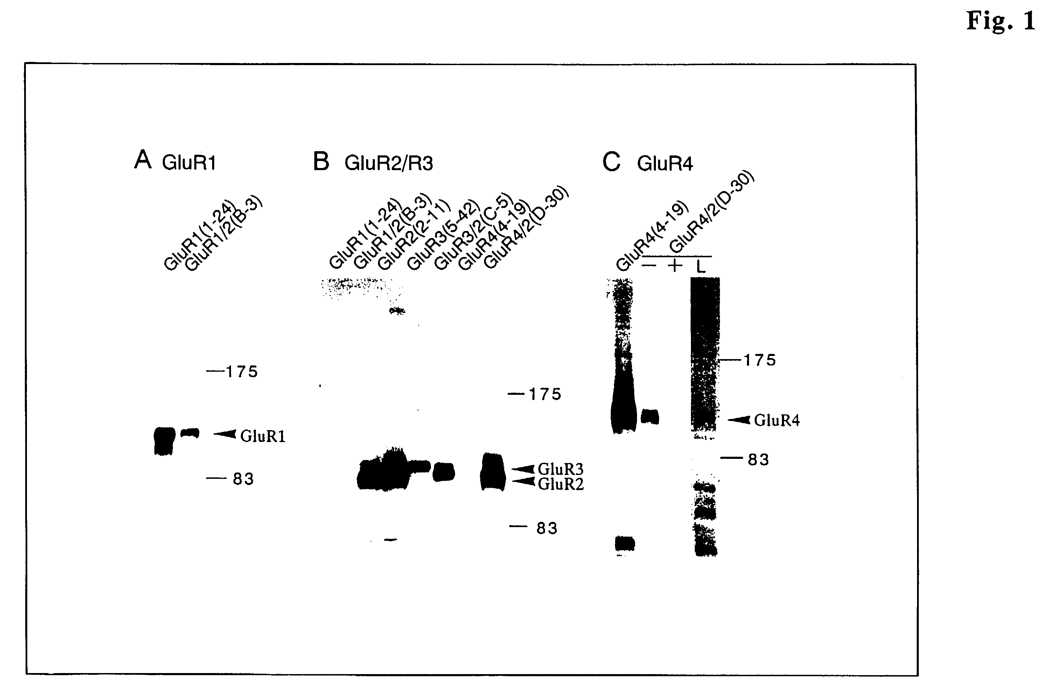

### Figure 1 Western blot of HEK293 cell lines stably expressing human AMPA receptor subunits

Twenty µg protein were loaded per lane. Expression of AMPA receptor subunits was investigated using anti-GluR1 (A), anti-GluR2/R3 (B) and anti-GluR4 (C) antibodies. In the inducible cell line GluR4/2 (D-30), expression of GluR4 subunit was investigated in the presence (+ ; suppressed) or absence (- ; induced) of tetracycline (Tet). Under the suppressed conditions, a leaky expression of GluR4 polypeptide (L) was detected by long photographic exposure (3-5 minutes), but not normal exposure period (15-30 seconds). The positions of each subunit and molecular weight markers are indicated at the right.

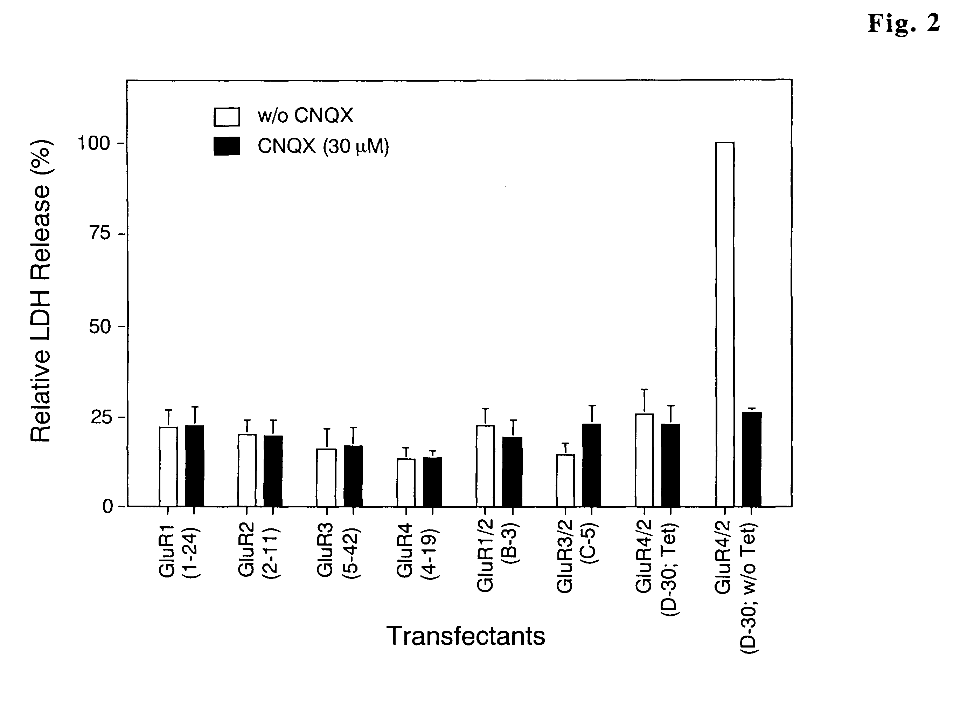

### Figure 2 Lethality of transfectants expressing human AMPA receptor subunits

Each of transformant cells (1 x 10⁵ seeded) was cultured for three days in the presence or absence of CNQX (30 µM), and collected for the LDH assay. GluR4/2 (D-30) cells were cultured in the presence or absence of tetracycline and CNQX. In all experiments, 100 µM of L-glutamic acid hydrochloride was added to the culture medium to activate constitutively the recombinant AMPA receptors expressed on the membrane of each transfectant. Data were normalized to the percent LDH release from GluR4/2 (D-30) cells cultured in the absence of tetracycline and CNQX. The basal LDH release of the GluR4/2 (D-30) cells was 44.4 ± 0.9 % (n=3). Each column and respective vertical bar represents the mean ± S.E. of 2-3 experiments carried out in duplicate.

### Figure 3 I-V relationships evoked by 100 µM AMPA

I-V relationships from transfectants stably expressing the human homomeric and heteromeric AMPA receptors. AMPA was added in the presence of 100 µM cyclothiazide. Each I-V curve was produced by the voltage ramp method.

### Figure 4 Agonist-evoked currents from transfectants stably expressing human homomeric and heteromeric AMPA receptors

(A) Glutamate (a)- and AMPA (b)-evoked currents for the GluR3 subunit and the combination of GluR1/ GluR2 subunits. Glutamate (0.01-1 mM) and AMPA (0.003-3 mM) were applied in the presence of 100 µM cyclothiazide. The cell was held at a membrane potential of -50 mV.
(B) Concentration-response curves of glutamate (a) or AMPA (b) in the presence of 100 µM cyclothiazide. All currents were normalized to the peak amplitudes induced by 3 mM AMPA. Each point represents the mean ± S.E. of 3-6 experiments.

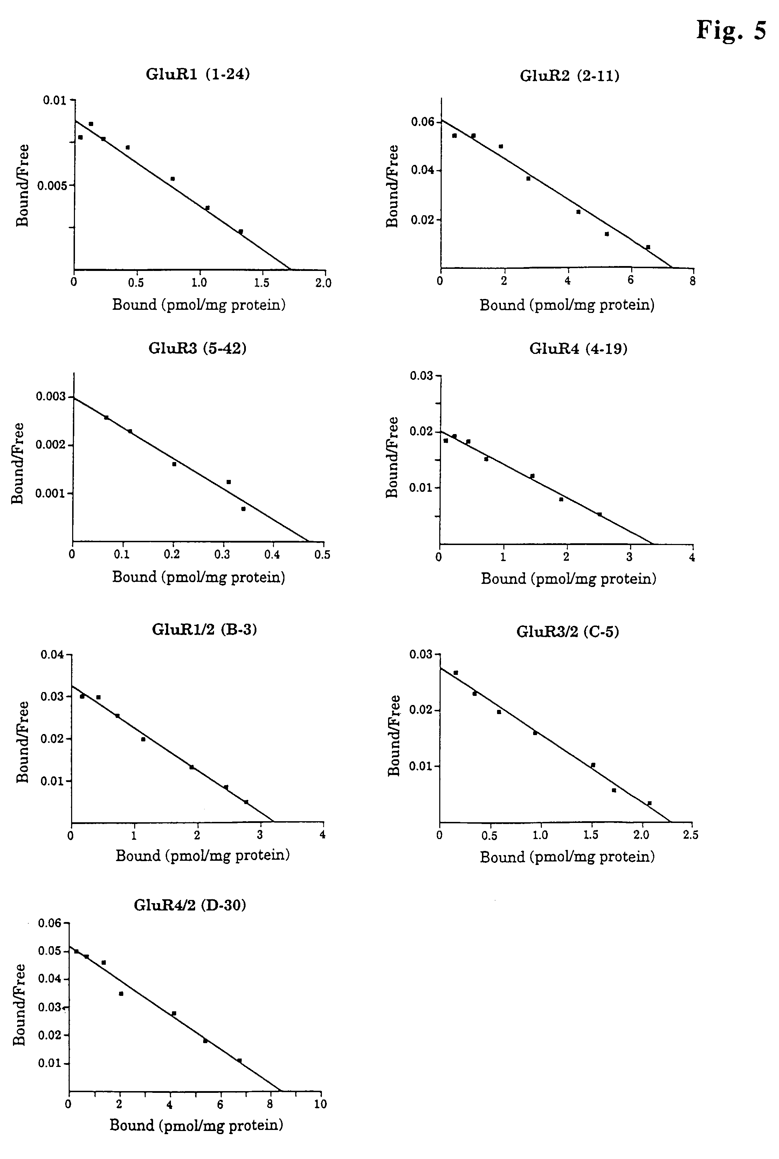

### Figure 5 Scatchard plots of [³H]AMPA binding

Scatchard analysis was performed in the concentration of 1 to 100 nM [³H]AMPA. All analyses were carried out in duplicate.

### Disclosure of the preferred embodiments of the invention

Before the embodiments of the present invention it must be noted that as used herein and in the appended claims, the singular forms "a", "an", and "the" include plural reference unless the context clearly dictates otherwise. Thus, for example, reference to "an AMPA receptor" includes a plurality of such AMPA receptors, reference to the "cell" is a reference to one or more cells and equivalents thereof known to those skilled in the art, and so forth. Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All publications mentioned herein are incorporated herein by reference for the purpose of describing and disclosing the cell lines, vectors, and methodologies which are reported in the publications which might be used in connection with the invention. Nothing herein is to be construed as an admission that the invention is not entitled to antedate such disclosure by virtue of prior invention. The present invention provides a new cell line stably expressing a recombinant heteromeric AMPA glutamate receptor on its cell surface.

The term "AMPA glutamate receptor" or "AMPA receptor" (both terms are used interchangeable) as used herein relates to non-NMDA receptors composed of glutamate receptor subunits (GluR). Of said glutamate receptor subunits, four (GluR1, GluR2, GluR3, GluR4, also termed GluR-A, GluR-B, GluR-C, GluR-D) contain binding sites that have higher affinity for AMPA than for kainate. Said glutamate receptor subunits are disclosed e.g. in Keinänen et al., 1990; Sommer et al., 1990; Hollmann et al., 1991; Verdoorn et al., 1991; Mosbacher et al., 1994. Splice variants or other functionally equivalent receptor subunits (e.g. carrying mutations in a single amino acid or several amino acids) are also included herein. They may be derived from any vertebrate species, preferably from mammalian species such as rat, mouse, hamster, pig, cattle, deer, horse and most preferably human. Thus, as understood herein, said AMPA receptors are comprising any combination of said GluR1-GluR4 subunits and preferably comprise a heterooligomer or homooligomer of said subunits. Said receptor subunits, when assembled together, form a central ion channel. A "heteromeric AMPA glutamate receptor" as used in the present invention refers to a receptor comprising at least two different subunits. There is insecurity in the art whether native GluR channels in hippocampal neurons are heterooligomers or a mosaic of homooligomers (Jonas, 1993, Jonas and Burnashev, 1995). It is speculated that all naturally occuring AMPA receptors, e.g. on primary neuronal cells, are heteromeric (Lees, 1996). Primary neuronal cells, however, as they are post-mitotic cells and have to be freshly prepared for every experiment, are not very useful in many research applications and in particular not in industrial applications such as HTS. The present invention now provides cell lines stably expressing heteromeric AMPA receptors which is a research tool most closely simulating the natural situation and thus overcomes the above-mentioned disadvantages in the prior art where only primary neuronal cells and transiently transfected cell lines are available.

Cells which have been "stably transformed" have recombinant DNA incorporated into their genomic DNA. Such stably incorporated DNA is retained by the transformed cells because it is introduced into the cells with a selection agent which forces retention when the cells are grown in a selection medium. The present invention preferably employs mammalian cell lines that have been stably transformed.

Said recombinant DNA is under control of a transcriptional initiation region (or promoter) which may be a constitutive promoter or an inducible or developmentally regulated promoter. Of particular interest but not exclusive are the constitutive promoters of the human cytomegalovirus (CMV) and Rous sarcoma virus (RSV), as well as the Simian virus 40 and Herpes simplex promoters. Useful inducible promoters include antibiotic resistant promoters, heat-shock promoters, the hormone-inducible mammary tumor promoter and the metallothionein promoter.

A suitable cell or cell line, preferably an eukaryotic cell or a cell line, to be transformed with nucleic acid constructs to express said expressing a recombinant heteromeric AMPA glutamate receptor on its cell surface may be any cell or cell line known to the expert in the field, in particular cells or cell lines used in neurological and neurobiology research. Examples of such cells or cell lines useful for producing the transformed cell lines of the invention include mammalian cells or cell lines (e.g. the cell lines human embryonic kidney (HEK) 293, BHK, GH3, H4, U373, NT2, PC12, COS, CHO, Ltk⁻, fibroblasts, myelomas, neuroblastomas, hybridomas, oocytes, embryonic stem cells), insect cell lines (e.g., using baculovirus vectors such as pPbac or pMbac (Stratagene, La Jolla, USA)), yeast (e.g., Pichia pastoris or using yeast expression vectors such as pYESHIS (Invitrogen, San Diego, USA)), and fungi.

In a more preferred embodiment, the invention relates to a cell line as described, wherein said receptor comprises the glutamate receptor 1 subunit (GluR1). As used in the present invention, GluR1 also relates to the glutamate receptor A subunit (GluR-A).

In another more preferred embodiment, the invention relates to a cell line as described, wherein said receptor comprises the glutamate receptor 2 subunit (GluR2). As used in the present invention, GluR2 also relates to the glutamate receptor B subunit (GluR-B).

In another more preferred embodiment, the invention relates to a cell line as described, wherein said receptor comprises the glutamate receptor 3 subunit (GluR3). As used in the present invention, GluR3 also relates to the glutamate receptor C subunit (GluR-C).

In another more preferred embodiment, the invention relates to a cell line as described, wherein said receptor comprises the glutamate receptor 4 subunit (GluR4). As used in the present invention, GluR4 also relates to the glutamate receptor D subunit (GluR-D).

In another more preferred embodiment, the invention relates to a cell line as described, wherein said receptor comprises of the glutamate receptor 1 (GluR1) and the glutamate receptor 2 subunit (GluR2).

In another more preferred embodiment, the invention relates to a cell line as described, wherein said receptor comprises of the glutamate receptor 1 (GluR1) and the glutamate receptor 3 subunit (GluR3).

In another more preferred embodiment, the invention relates to a cell line as described, wherein said receptor comprises of the glutamate receptor 1 (GluR1) and the glutamate receptor 4 subunit (GluR4).

In another more preferred embodiment, the invention relates to a cell line as described, wherein said receptor comprises of the glutamate receptor 2 (GluR2) and the glutamate receptor 3 subunit (GluR3).

In another more preferred embodiment, the invention relates to a cell line as described, wherein said receptor comprises of the glutamate receptor 2 (GluR2) and the glutamate receptor 4 subunit (GluR4).

In another more preferred embodiment, the invention relates to a cell line as described, wherein said receptor comprises of the glutamate receptor 3 (GluR3) and the glutamate receptor 4 subunit (GluR4).

A functional AMPA glutamate receptor structure possibly comprises a heterooligomer of five subunits wherein at least two of said subunits are different.

Thus, in another more preferred embodiment, the invention relates to a cell line as described, wherein said receptor comprises five glutamate receptor (GluR) subunits according to the description *supra.*

As described *supra,* a disadvantage of the prior art is that cell lines expressing non-human AMPA-receptors cannot be used to investigate the function of human AMPA receptors or human neurological disorders associated with said receptors. This disadvantage of the prior art is overcome with the cell line of the present invention. Thus, in a preferred embodiment, the invention relates to a cell line as described, wherein said cell line expresses a recombinant human AMPA glutamate receptor. Said cell line stably expressing a human AMPA receptor can be a more precise, species-targeted tool for the identification of novel drugs useful in the therapy of neurological diseases and post-ischemic neurocytotoxic conditions.

A further aspect of the present invention is a cell line stably expressing a recombinant human homomeric AMPA glutamate receptor wherein said receptor comprises at least two glutamate receptor 1 (GluR1) subunits.

A "homomeric AMPA glutamate receptor" as used in the present invention refers to a receptor comprising only one type of subunit.

A still further aspect of the present invention is a cell line stably expressing a recombinant human homomeric AMPA glutamate receptor wherein said receptor comprises at least two glutamate receptor 2 subunits (GluR2).

A still further aspect of the present invention is a cell line stably expressing a recombinant human homomeric AMPA glutamate receptor wherein said receptor comprises at least two glutamate receptor 3 subunits (GluR3).

A still further aspect of the present invention is a cell line stably expressing a recombinant human homomeric AMPA glutamate receptor wherein said receptor comprises at least two glutamate receptor 4 subunits (GluR4). Said cell lines of the present invention expressing a human homomeric AMPA receptor can, in a similar manner as described *supra* for cell lines stably expressing a human heteromeric AMPA glutamate receptor, overcome the disadvantages in the prior art and can be a more precise, species-targeted tool for the identification of novel drugs useful in the therapy of neurological diseases and post-ischemic neurocytotoxic conditions.

Another important aspect of the present invention is a cell line as described *supra,* wherein said receptor comprises five glutamate receptor subunits (GluR). Said five subunits are either five GluR1 or five GluR2 or five GluR3 or five GluR4 subunits.

As disclosed *supra,* each AMPA receptor subunit exists in two different forms, designated "flip" and "flop", created by alternative splicing of a 115-base pair region preceding the transmembrane domain IV.

Thus, another important aspect of the present invention is a cell line as described *supra,* wherein said receptor comprises the flip splice variant of the subunit.

Another important aspect of the present invention is a cell line as described *supra,* wherein said receptor comprises the flop splice variant of the subunit.

Yet another important embodiment of the present invention is a cell line stably expressing a recombinant human homomeric AMPA glutamate receptor wherein said receptor comprises at least two flop splice variants of the glutamate receptor 3 subunit (GluR3).

Yet another important embodiment of the present invention is a cell line stably expressing a recombinant human homomeric AMPA glutamate receptor wherein said receptor comprises at least two flop splice variants of the glutamate receptor 4 subunit (GluR4).

A most preferred embodiment of the present invention is a cell line as described *supra,* which is derived from the HEK 293 cell line.

Another most preferred embodiment of the present invention is a HEK 293 cell line stably expressing a recombinant human homomeric AMPA glutamate receptor on its cell surface, wherein said receptor consists of the flip splice variant of the glutamate receptor 1 (GluR1).

Another most preferred embodiment of the present invention is a HEK 293 cell line stably expressing a recombinant human homomeric AMPA glutamate receptor on its cell surface, wherein said receptor consists of the flip splice variant of the glutamate receptor 2 (GluR2).

Another most preferred embodiment of the present invention is a HEK 293 cell line stably expressing a recombinant human homomeric AMPA glutamate receptor on its cell surface, wherein said receptor consists of the flip splice variant of the glutamate receptor 3 (GluR3).

Another most preferred embodiment of the present invention is a HEK 293 cell line stably expressing a recombinant human homomeric AMPA glutamate receptor on its cell surface, wherein said receptor consists of the flip splice variant of the glutamate receptor 4 (GluR4).

Another most preferred embodiment of the present invention is a HEK 293 cell line stably expressing a recombinant human heteromeric AMPA glutamate receptor on its cell surface, wherein said receptor consists of the flip splice variants of the glutamate receptor 1 (GluR1) and the glutamate receptor 2 subunits (GluR2).

Another most preferred embodiment of the present invention is a HEK 293 cell line stably expressing a recombinant human heteromeric AMPA glutamate receptor on its cell surface, wherein said receptor consists of the flip splice variants of the glutamate receptor 2 (GluR2) and the glutamate receptor 3 subunits (GluR3).

Another most preferred embodiment of the present invention is a HEK 293 cell line stably expressing a recombinant human heteromeric AMPA glutamate receptor on its cell surface, wherein said receptor consists of the flip splice variants of the glutamate receptor 2 (GluR2) and the glutamate receptor 4 subunits (GluR4).

Surprisingly, said cell lines of the present invention possess functional AMPA channels on the basis of electrophysiological and pharmacological properties which is also - in a non-limiting manner - demonstrated in examples 2 and 3.

Another important aspect of the present invention is a method for preparing a cell line as described *supra* comprising the construction of an expression vector which comprises DNA specific for a glutamate receptor subunit, the transfection of a suitable cell line with said expression vector and the screening said cell line for stable transfection employing a suitable selection agent.

For the purpose of the present invention, DNA encoding an AMPA receptor or a subunit thereof can be obtained from any cDNA library prepared from tissue believed to possess the AMPA receptor mRNA and to express it at a detectable level. For example, a human brain cDNA library, such as that described in the examples, is a good source of AMPA receptor or AMPA receptor subunit cDNA. The AMPA receptor or AMPA receptor subunit genes can also be obtained from a genomic library, such as a human genomic cosmid library. The sequences of human glutamate receptor subunits are publicly available e.g. in the database Gene Bank: hGluR1 accession number M64752; hGluR2 L20814; hGluR3 U10301 and hGluR4 U16129. The cDNA encoding the AMPA receptor or AMPA receptor subunit cDNA may be then used for the ligation into appropriate vectors and transformation of appropriate cells or cell lines with said vectors (a non-limiting example of the invention see example 1).

Methods of producing appropriate recombinant vectors, transforming cells with those recombinant vectors, and identifying transformants are well known in the art and are only briefly reviewed here (see, for example, Sambrook et al. 1989).

Suitable vectors comprise plasmids, viruses (including phages) and integratable DNA fragments (i.e., integratable into the host genome by recombination). In the present specification, "vector" is generic to "plasmid"; but plasmids are the most commonly used form of vectors at present. However, all other forms of vectors which serve an equivalent function and which are, or become, known in the art are suitable for use herein. Suitable vectors will contain replicon and control sequences which are derived from species compatible with the intended expression host.

Suitable host cells are any prokaryotes, yeasts or higher eukaryotic cells which have been transformed or transfected with the nucleic acids of the present invention so as to cause clonal propagation of those nucleic acids and/or expression of the proteins or peptides encoded thereby. Such cells or cell lines will have utility both in the propagation and production of the nucleic acids and proteins of the present invention but also, as further described herein, as model systems for diagnostic and therapeutic assays. As used herein, the term "transformed cell" is intended to embrace any cell, or the descendant of any cell, into which has been introduced any of the nucleic acids of the invention, whether by transformation, transfection, infection, or other means.

Prokaryotic cells useful for producing the transformed cells of the invention include members of the bacterial genera Escherichia (e.g., E. coli), Pseudomonas (e.g., P. aeruginosa), and Bacillus (e.g., B. subtillus, B. stearothermophilus), as well as many others well known and frequently used in the art. Prokaryotic cells are particularly useful for the production of large quantities of the receptor proteins of the invention (e.g. normal or mutant AMPA glutamate receptors or subunits thereof, fragments of the said receptors or subunits, fusion proteins of said receptors or subunits). Bacterial cells (e.g., E. coli) may be used with a variety of expression vector systems including, for example, plasmids with the T7 RNA polymerase/promoter system, bacteriophage λ regulatory sequences, or M13 Phage mGPI-2. Bacterial hosts may also be transformed with fusion protein vectors which create, for example, lacZ, trpE, maltose-binding protein, poly-His tags, or glutathione-S-transferase fusion proteins. All of these, as well as many other prokaryotic expression systems, are well known in the art and widely commercially available (e.g., pGEX-27 (Amrad, USA) for GST fusions).

Eukaryotic cells and cell lines useful for producing the transformed cells of the invention are described *infra.*

To accomplish expression in eukaryotic cells, a wide variety of vectors have been developed and are commercially available which allow inducible (e.g., LacSwitch expression vectors, Stratagene, La Jolla, CA) or cognate (e.g., pcDNA3 vectors, Invitrogen, San Diego, USA) nucleotide sequence expression of AMPA glutamate receptor subunits or entire receptors under the regulation of an artificial promoter element. Such promoter elements are often derived from CMV or SV40 viral genes, although other strong promoter elements which are active in eukaryotic cells can also be employed to induce transcription of expression of AMPA glutamate receptor subunit or entire receptor nucleotide sequences. Typically, these vectors also contain an artificial polyadenylation sequence and 3' UTR which can also be derived from exogenous viral gene sequences or from other eukaryotic genes. Furthermore, in some constructs, artificial, noncoding, spliceable introns and exons are included in the vector to enhance expression of the nucleotide sequence of interest (in this case, AMPA glutamate receptor subunit or entire receptor nucleotide sequences). These expression systems are commonly available from commercial sources and are typified by vectors such as pcDNA3 and pZeoSV (Invitrogen, San Diego, USA). Innumerable commercially-available as well as custom-designed expression vectors are available from commercial sources to allow expression of any desired expression of AMPA glutamate receptor subunit or entire receptor transcript in more or less any desired cell type, either constitutively or after exposure to a certain exogenous, stimulus (e.g., withdrawal of tetracycline or exposure to IPTG).

Recombinant vectors may be introduced into the recipient or "host" cells by various methods well known in the art including, but not limited to, calcium phosphate transfection, strontium phosphate transfection, DEAE dextran transfection, electroporation, lipofection (e.g., Dosper Liposomal transfection reagent, Boehringer Mannheim, Germany), microinjection, ballistic insertion on micro-beads, protoplast fusion or, for viral or phage vectors, by infection with the recombinant virus or phage.

Thus, stable transformation of a mammalian cell line may be accomplished by using standard methods to co-transfect the cells with one or several recombinant vectors comprising nucleic acid molecules encoding an AMPA glutamate receptor subunit or entire receptor according to the present invention and with a second vector which confers resistance to a selection agent such as an antibiotic, e. g. G418 (neomycin) or Zeocin. Alternatively, transformation can be carried out with a single vector containing both the genetic control element and the selection agent gene.

Recombinant retroviral vectors can also contain a selection agent gene to produce stable transformation. To co-transfect cells to express heteromeric AMPA glutamate receptor according to the present invention, a different selection agent for every AMPA glutamate receptor subunit cDNA may be used. Example 1, which should not be construed as to limiting the present invention, exemplifies e.g. co-transfection of HEK293 cells with a plasmid comprising the GluR1 AMPA receptor subunit cDNA linked to the gene for neomycin resistance (pHGluR1/pcDNA3) and a plasmid comprising the GluR2 AMPA receptor subunit cDNA linked to the gene for Zeocin resistance (pHGluR2/pcDNA3.1/Zeo(+)).

Thus, another important aspect of the present invention is a method as described, wherein said cell line is co-transfected with two or more of said expression vectors each of which is comprising DNA specific for a different glutamate receptor subunit.

The cell lines according to the present invention, preferably the cell lines expressing the human AMPA homomeric or even more preferred, the heteromeric receptor constitute valuable tools for the artisan. Said cell lines of the present invention are more precise, species-targeted tools for identification and characterization of new AMPA receptor modulatory ligands.

Thus, another important embodiment of the present invention is the use of a cell line according to the description *supra* for the identification of glutamate receptor agonists.

Yet another important embodiment of the present invention is the use of a cell line according to the description *supra* for the identification of glutamate receptor antagonists or modulators.

As used herein, "glutamate receptor" refers to any type of ionotropic receptor, preferably the N-methyl-D-aspartate (NMDA) receptors, the a-amino-3-hydroxy-5-methyl-4-isoxazolepropionate (AMPA) receptors, the kainate receptors, and the metabotropic receptors.

The term "agonist", as used herein, refers to a substance or signal, that activates AMPA receptor function; and the terms "antagonist" or "modulator" refer to a substance that interferes with AMPA receptor function.

Agonists include, but are not limited to chemical compounds such as small organic molecules, proteins, nucleic acids, carbohydrates, or any other molecules which bind to AMPA receptors. Examples for known AMPA receptor agonists are AMPA, kainate, BOAA, domoate, Acromelate A and glutamate.

The terms "antagonist" or "modulator" as used herein, refer to a molecule which, when bound to AMPA receptors, blocks or modulates the biological or neurological activity of AMPA receptors e.g. by blocking the activation of the AMPA receptor through agonists. Modulators as used herein refer e.g. to allosteric modulators. Antagonists or modulators include competitive and non-competitive antagonists or modulators. A competitive antagonist (or competitive blocker) interacts with or near the site specific for the agonist (e.g., an AMPA receptor ligand) for the same or closely situated site. A non-competitive competitive antagonist or modulator or blocker inactivates the functioning of the receptor by interacting with a site other than the site that interacts with the agonist. Antagonists or modulators include, but are not limited to chemical compounds such as small organic molecules, proteins, nucleic acids, carbohydrates, or any other molecules which bind to AMPA receptors. Examples for known AMPA receptor antagonists or modulators are CNQX, NBQX, YM90K, (+) LY 215490, (-) LY 215490, NS102, NS257 and cyclothiazide.

Yet another important embodiment of the present invention is the use of a cell line according to the description *supra* in a high throughput screening (HTS) format. HTS relates to an experimental setup wherein a large number of compounds is tested simultaneously. Preferably, said HTS setup may be carried out in microplates, may be partially or fully automated and may be linked to electronic devices such as computers for data storage, analysis, and interpretation using bioinformatics. Preferably, said automation may involve robots capable of handling large numbers of microplates and capable of carrying out several thousand tests per day. Preferably, a test compound which shows a desired agonist, antagonist or modulator function in a cell-free system will also be tested in a cell-based system using a cell line according to the present invention. The term HTS also comprises ultra high throughput screening formats (UHTS). Preferably, said UHTS formats may be carried out using 384- or 1536-well microplates, sub-microliter or sub-nanoliter pipettors, improved plate readers and procedures to deal with evaporation. HTS methods are described e.g. in US 5876946 A or US 5902732 A. The expert in the field can adapt the method described below to a HTS or UHTS format without the need of carrying out an inventive step.

Yet another important embodiment of the present invention is the use of a cell line according to the description *supra* in an electrophysiological assay with an agonist, antagonist or modulator of a glutamate receptor wherein membrane currents are measured. Electrophysiological assays are known to the artisan and are disclosed in e.g. Hille, B., 1984. A non-limiting example of such an assay is disclosed in example 2.

Yet another important embodiment of the present invention is the use of a cell line according to the description *supra* in a receptor-binding assay (see description *infra).*

Yet another important embodiment of the present invention is the use of a cell line according to the description *supra* in a fluorometric assay (see description *infra)* wherein the intracellular quantity of a divalent ion is measured.

In a further important aspect, the present invention relates to a method for the identification of an agonist, antagonist or modulator of a glutamate receptor comprising the incubation of a cell line as described *supra* with a test substance.

In another important aspect, the invention relates to a method as described, wherein the method is further characterized in that the measurement of a membrane current and the comparison of said membrane current with the membrane current obtained for said cell line after incubation with a known control or in the absence of the test substance.

As understood by those of skilled in the art, methods for the identification of an agonist, antagonist or modulator of a glutamate receptor generally require comparison to a control. One type of a "control" cell or "control" culture is a cell or culture that is treated substantially the same as the cell or culture exposed to the test compound, except the control culture is not exposed to test compound. For example, in methods that use voltage clamp electrophysiological procedures, the same cell can be tested in the presence and absence of test compound, by merely changing the external solution bathing the cell. Another type of "control" cell or "control" culture may be a cell or a culture of cells which are identical to the transfected cells, except the cells employed for the control culture do not express the AMPA glutamate receptor expressed in the stably transfected cells according to the invention. In this situation, the response of test cell to test compound is compared to the response (or lack of response) of receptor-negative (control) cell to test compound, when cells or cultures of each type of cell are exposed to substantially the same reaction conditions in the presence of compound being assayed. The above-mentioned known agonists, antagonists or modulators may also be used as a control for the methods of the present invention. In this case, cells or cultures of the cell lines according to the invention are incubated with the test substance, in a parallel experiment cells or cultures of said cell lines are incubated with a known agonist, antagonist or modulator and both responses are compared.

Said methods according to the invention may be biochemical, molecular biology or immunological methods. Biochemical or molecular biology methods are known to the expert in the field and include, but are not limited to: reporter gene assays such as β-gal-, CAT-, SEAP- GFP-, BFP- or luciferase-assays, polymerase-chain reaction (PCR), RT-PCR, Northern- or Southern-blots which are published e.g. in: Sambrook et al.(1989) Molecular Cloning: A Laboratory Manual, 2^{nd} ed., Cold Spring Harbor Laboratory Press, Cold Spring Harbor, New York and Bertram, S. and Gassen, H.G. Gentechnische Methoden, G. Fischer Verlag, Stuttgart, New York, 1991). Immunological methods are known to the expert in the field and include, but are not limited to ELISAs (***e**nzyme-**l**inked **i**mmuno-**s**orbent assay*) or *Sandwich*-ELISAs, *dot-blots*, immunoblots, radioimmunoassays (***R**adio**i**mmuno**a**ssay RIA*), diffusion-based Ouchterlony tests, rocket immunofluorescent assays or Western-blots. Examples for immunological methods are e.g. described in: *An Introduction to Radioimmunoassay and Related Techniques,* Elsevier Science Publishers, Amsterdam. The Netherlands (1986); Bullock *et al., Techniques in Immunocytochemistry,* Academic Press, Orlando, FL Vol. 1 (1982), Vol. 2 (1983), Vol. 3 (1985); Tijssen, *Practice and Theory of Enzyme Immunoassays: Laboratory Techniques in Biochemistry and Molecular Biology,* Elsevier Science Publishers, Amsterdam, The Netherlands (1985).

Furthermore, the methods according to the present invention preferably include electrophysiological methods or assays as described *supra,* e.g. a patch-clamp method (see also e.g. Mayer, ML et al., 1989). The artisan knows numerous methods which can be used for the identification of agonists, antagonists or modulators of AMPA receptors and can adapt them without carrying out an inventive step to include a cell line according to the present invention (see e.g. Hille, B., 1984). Examples 2 and 3 disclose non-limiting examples of the methods according to the present invention, however, said methods are only carried out with the known agonists AMPA, glutamate or the antagonist CNQX, respectively, and not with test compounds. However, test compounds, e.g. libraries containing small organic molecules, are widely available from commercial sources and can be included in the methods according to the present invention.

In another preferred aspect, the invention relates to a method as described, wherein said method is characterized in that a cell line as described *supra* is incubated with a known agonist which is coupled to a reporter, said cell line is incubated with a test substance, the displacement of the agonist coupled to the reporter by the test substance is measured. All above-mentioned known agonists can be used in this preferred method and can be coupled to a reporter without inventive step. A reporter as used herein may be any reporter known to the artisan and will depend upon the type of method used. Examples of reporters that can be used include, e.g., radiolabels such as ³²P, ¹²⁵I, ³H and ¹⁴C; fluorescent reporters such as fluorescein and its derivatives, rhodamine and its derivatives, dansyl and umbelliferone and chemiluminescers such as the various luciferin compounds. For example, this method may be a receptor-binding assay, wherein e.g. the known agonist AMPA is coupled to the reporter tritium [³H] and the displacement of [³H]AMPA is measured. A non-limiting example of this method is disclosed in example 3.

In another preferred aspect, the invention relates to a method as described, wherein said method is characterized in that a cell line as described *supra* is incubated with a test substance, the intracellular quantity of a divalent cation is measured, said quantity of the intracellular divalent cation (e.g. calcium, magnesium, barium) is compared with the quantity of the intracellular divalent ion measured for said cell line after incubation with a known control or in the absence of the test substance. The above-mentioned known agonists, antagonists or modulators can be used as a control for this preferred method of the present invention. Said method may be a fluorometric or colorimetric method wherein e.g. intracellular calcium ions are measured. For the measurement of intracellular calcium concentrations, there are sensitive and relatively simple assays known in the art which use either calcium-chelating fluorescent dyes or calcium-binding bioluminescent proteins. Calcium-chelating fluorescent dyes such as Fura-2, Fluo-3, Indo-1 (Molecular Probes, Inc., Eugene, USA), Quin-2 are well-established tools that have been employed in studies of voltage-sensitive calcium channels (Tsien, 1988). Also suitable are the calcium indicators described in U.S. Pat. No.5,453,517 to Kuhn et al. (1995). Selected embodiments of these indicators are sold under the trademarks Calcium Green, Calcium Orange and Calcium Crimson (Molecular Probes, Inc.). Also suitable are the long-wavelength calcium indicators described in U.S. Pat. No. 5,501,980 to Katerinopoulos et al. (1996). A specific embodiment of these indicators is sold under the name BTC (Molecular Probes, Inc.). An additional useful fluorescent calcium indicator has been described in U.S. Pat. No. 4,849,362 to DeMarinis et al. (1989) and is sold under the trademark Fura Red (Molecular Probes, Inc.) and calcium ion indicators with enhanced photostability sold under the trademark Oregon Green Bapta indicators (Molecular Probes, Inc.).

Said calcium-sensitive indicators, such as Fluo-3 and Fura-2 are available as acetoxymethyl esters which are membrane permeable. When the acetoxymethyl ester form of the indicator enters a cell, the ester group is removed by cytosolic esterases, thereby trapping the free indicator in the cytosol. Interaction of the free indicator with calcium results in increased fluorescence of the indicator; therefore, an increase in the intracellular Ca⁺⁺ concentration of cells containing the indicator can be expressed directly as an increase in fluorescence (or an increase in the ratio of the fluorescence at two wavelengths when Fura-2 is used). A variety of divalent cation indicators, ion probes and assays for selected cellular functions are described by Haugland (Molecular probes. Handbook of fluorescent probes and research chemicals, sets 20-23 and 25, 1992-94).

Alternatively, calcium influx may be assessed using the bioluminescent properties of the calcium-binding protein aequorin. Expression of the cloned aequorin gene (Prasher et al. 1985) in E. coli (Knight et al. 1991) or cultured human cells (Sheu et al. 1993) has been shown to provide a sensitive luminescent assay for changes in intracellular calcium concentration.

In a further preferred aspect, the present invention relates to a method as described, wherein said method is a high throughput screening assay (HTS; see description *supra*).

Another preferred embodiment of the present invention is a pharmaceutical composition comprising an agonist antagonist or modulator of a glutamate receptor identifiable with a method as described *supra* and a pharmaceutically acceptable carrier.

A pharmaceutically acceptable carrier can contain physiologically acceptable compounds that act, for example, to stabilize or to increase the absorption of an AMPA glutamate receptor agonist, antagonist or modulator. Such physiologically acceptable compounds include, for example, carbohydrates, such as glucose, sucrose or dextrans, antioxidants, such as ascorbic acid or glutathione, chelating agents, low molecular weight proteins or other stabilizers or excipients (see also e.g. Remington's Pharmaceutical Sciences (1990)). One skilled in the art would know that the choice of a pharmaceutically acceptable carrier, including a physiologically acceptable compound, depends, for example, on the route of administration of the composition.

Another preferred embodiment of the present invention is the use of an agonist or antagonist of a glutamate receptor identifiable with a method according to the present invention in the manufacture of a medicament for the treatment of neurological disorders. Neurological disorders include, but are not limited to disorders in which a neurotoxic action of glutamate itself or other glutamate receptor agonists may be involved such as global and focal ischaemia, ischaemic and haemorrhagic stroke, global cerebral ischaemia with heart stoppage, post-ischaemic neurocytotoxic conditions, hypoglycaemia, diabetic polyneuropathy, hypoxia, anoxia, physical trauma, brain trauma, brain edema, brain pressure (elevated intracranial pressure), hypotonia, cardiac infarction, drug abuse with amphetamine and related compounds, metabolic poisoning such as with carbon monoxide, certain food toxicities (e.g. shellfish poisoning, neurolathyrism), epilepsy (e.g. kainate induced status epilepticus), AIDS-related dementia, tinnitus, perinatal asphyxia, psychosis, schizophrenia, depression, motor neuron disease (including amyotrophic lateral sclerosis) and furthermore include neurodegenerative diseases such as Alzheimer's disease, Parkinson's disease, ataxia such as spinocerebellar ataxia, Kennedy disease and Huntigton's chorea.

The following examples serve to further illustrate the present invention; but the same should not be construed as limiting the scope of the invention disclosed herein.

### Examples

### Example 1 - Establishment of stable transfectants expressing the human homomeric and the heteromeric AMPA receptor subunits

Stably transfected cell lines expressing the human AMPA receptors, GluR1 (1-24), GluR2 (2-11), GluR3 (5-42) and GluR4 (4-19), were established in HEK293 cells. Transformants stably expressing the heteromeric AMPA receptors, GluRl/2 (B-3) and GluR3/2 (C-5), were obtained by the simultaneous co-transfection of HEK293 cells with two different expression-plasmids containing each subunit cDNA, respectively. However, it was not possible to establish transfectants stably co-expressing GluR4 and GluR2 subunits using the same method. In some isolated clones, expression of GluR2 subunit was only detectable in western blot analysis, but not GluR4 expression, and their electrophysiological profiles showed linear I-V relationships, in which the current amplitudes were small (data not shown). To circumvent this problem, the transfectant GluR4/2 (D-30) was generated by introducing the tetracycline-regulated inducible plasmid containing GluR4 subunit cDNA into GluR2 (2-11) cells stably expressing GluR2 subunit.

The expression of AMPA receptor subunits in transfected cell lines was investigated by immunochemical analysis (Fig. 1). The antibodies specific to GluR1, GluR2/GluR3, and GluR4 subunits recognized a band of approximately 100 kDa, respectively. Anti-GluR2/R3 antibody, which recognizes the C-terminal amino acid sequences of both GluR2 and GluR3 subunits, distinguished these subunits according to the difference in their molecular sizes. The band for GluR3 exhibited a slightly higher molecular weight compared to GluR2. In noninducible expression conditions of GluR4/2 (D-30) cells, a faint GluR4 polypeptide was recognized, due to the inherent leaky character of the tetracycline-promoter, on a background of constitutive GluR2 expression. The GluR4 expression was markedly induced by removing tetracycline from the culture medium. The increased expression of GluR4 subunit yielded cell death which was determined on the third day after tetracycline removal using the cytotoxicity assay, which measures cell death by the activity of lactate dehydrogenase (LDH) released to the cell culture medium [GluR4/2 (D-30; w/o Tet) in Fig. 2]. The cell death was prevented by the inclusion of AMPA receptor antagonist CNQX. No specific lethality was apparent in the other clones expressing the homomeric (including GluR2 alone and GluR4 alone) and the heteromeric AMPA receptor subunits. In order to characterize the transfectant co-expressing GluR2 and GluR4 subunits, we used the GluR4/2 (D-30) clone in the continuous presence of tetracycline.

### 1. Cloning and expression constructs

### 1.1 Construction of expression plasmids

Expression plasmids pHGluR1/pcDNA3, pHGluR2/pcDNA3.1/Zeo(+), pHGluR3/pcDNA3 and pHGluR4/pcDNA3 carrying the human GluR1, GluR2, GluR3 and GluR4 subunit cDNA, respectively, were constructed as follows: all subunit cDNAs were derived from RT-PCR. One µg human brain poly (A)⁺ RNA (Clontech) was subjected to Moloney's murine leukemia virus RNase H-reverse transcriptase (GIBCO BRL) with random primers. The synthesized first strand cDNA was amplified with a DNA Thermal Cycler (Perkin-Elmer Corp.), according to the manufacture's specifications (TaKaRa LA PCR kit). Following the hot start (1 minute at 94°C), the samples were subjected to 30 cycles of 20 seconds at 98°C, and 5 minutes at 68°C.

Primer pairs for human GluR1 were synthetic 26-nucleotide oligomers at bases 26-51 (upstream primer, CAAATCTAAGCTTGGACCTGGGCTTC) and 2792-2817 (downstream primer, GATTGTAACTGGAGCAGATGGATCCC). The numbering of nucleotides is according to sequences derived from Gene Bank (HGluR1: accession number M64752; HGluR2: L20814; HGluR3: U10301; HGluR4: U16129). The reverse transcriptase PCR product of human GluR1 was ligated into pCR™2.1 vector (Invitrogen) to yield pHGluR1/pCR2.1. The 2.8 kilobase pair (kb) HindIII (vector)/BamHI (vector) fragment from pHGluR1/pCR2.1 was ligated into the HindIII/BamHI site of pcDNA3 expression vector (Invitrogen) which has a CMV promoter, to yield pHGluR1/pcDNA3. Primer pairs for human GluR2 were synthetic 25-nucleotide oligomers at bases 10 to 34 (upstream primer, CTGCCTCCACTTCAGGTTTTAGCAG) and 3301-3325 (downstream primer, ATCAACTGCATCAAGATGTGACATG). The reverse transcriptase PCR product of human GluR2 was blunted by T4 DNA polymerase and then cleaved by XhoI. The blunt end (10)/XhoI(3156) fragment from the PCR product was inserted into the EcoRV/XhoI site of pBluescript SK⁻ (Stratagene) to yield pHGluR2/BS. The 3.1 kb NotI(vector)/XhoI(3156) fragment from pHGluR2/BS was ligated into the NotI/XhoI site of pcDNA3.1/Zeo (+) expression vector (Invitrogen) which has a CMV promoter, to yield pHGluR2/pcDNA3.1/Zeo(+). The primer pairs for human GluR3 were synthetic 25-nucleotide oligomer at bases 1-25 (upstream primer, TGACGACTCCTGAGTTGCGCCCATG) and synthetic 20-nucleotide oligomer at bases 2741-2760 (downstream primer, ATCCCTTCCCACTGGAGGCA). The reverse transcriptase PCR product of human GluR3 was ligated into pCR™2.1 vector to yield pHGluR3/pCR2.1. The 2.8 kb KpnI(vector)/NotI(vector) fragment from pHGluR3/pCR2.1 was ligated into the KpnI/NotI site of pcDNA3 to yield pHGluR3/pcDNA3. Primer pairs for human GluR4 were synthetic 26-nucleotide oligomers at bases 1-26 (upstream primer, ATGAGGATTATTTCCAGACAGATTGT) and 2684-2709 (downstream primer, CTGTCATTGCATCGGACCTACCATAA). The reverse transcriptase PCR product of human GluR4 was ligated into pCR™2.1 vector to yield pHGluR4/pCR2.1. The plasmid pcDNA3 was cleaved by EcoRV, and ligated with the 2.7 kb SpeI (vector)/NotI (vector) fragment from pHGluR4/pCR2.1 which was blunted by T4 DNA polymerase, to yield pHGluR4/pcDNA3. For generation of inducible transfectant, the 2.7 kb XhoI (vector)/HindIII (vector) fragment from pHGluR4/pCR2.1 was ligated into the SalI/HindIII site of the tTA-dependent expression vector pAHygTet containing a CMV minimal promoter and 7 tet operators under control of tTA and tetracycline (Gossen and Bujard, 1992), to yield pHGluR4/pAHygTet. The reverse transcriptase PCR products were sequenced by dideoxy-chain-termination method.

### 1.2. Isolation of transfectants

Clones of GluR1 (1-24), GluR3 (5-42) and GluR4 (4-19) were obtained from HEK293 cells transformed with the plasmids pHGluR1/pcDNA3, pHGluR3/pcDNA3 and pHGluR4/pcDNA3, respectively. The G418 selected clones were characterized by RNA blotting using the cDNA of human GluR1, GluR3 and GluR4 subunit, respectively, as probes. The clone GluR2 (2-11) was isolated by transfecting HEK293 cells with the expression plasmid pHGluR2/pcDNA3.1/Zeo(+). The clone GluR2 (2-11) was selected with Zeocin, and characterized by RNA blotting using the cDNA of human GluR2 subunit as probe. The clone GluR1/2 (B-3) was isolated by cotransfecting HEK293 cells with the expression plasmids pHGluR1/pcDNA3 and pHGluR2/pcDNA3.1/Zeo(+). The clone GluR3/2 (C-5) was isolated by cotransfecting HEK293 cells with the expression plasmids pHGluR2/pcDNA3.1/Zeo(+) and pHGluR3/pcDNA3. The clones GluR1/2(B-3) and GluR3/2 (C-5) were selected with G418 and Zeocin, and characterized by RNA blotting using as probes the cDNAs of human GluR1, GluR2 and GluR3 subunits. The cells were maintained in DMEM medium (ICN) supplemented with 10% fetal bovine serum. Furthermore, the clone GluR4/2 (D-30) was isolated by cotransfecting GluR2 (2-11) cells with the expression plasmids pHGluR4/pAHygTet and pUHD15-1_{Neo}. This clone was normally maintained in the presence of 2 µg/ml tetracycline to suppress GluR4 expression.

### 2. Western blot analysis

Transfected cells were collected and homogenized in ice-cold Tris buffer (pH 7.0) containing 1 mM EDTA, 50 mM NaCl and 1 mM phenylmethylsulfonyl fluoride. Homogenates were centrifuged and membrane fractions were prepared. Aliquots of cell membrane (20 µg of protein) were subjected to MULTIGEL 7.5 (Daiichi pure chemical) and transferred to the GVHP filter (Millipore) by electroblotting (200 mA, 1.5 hours). Subsequent steps for blocking and detections were carried out using the ECL western blotting analysis (Amersham Life Science). As primary antibodies, anti-rat GluR1, anti-rat GluR2/R3 and anti-rat GluR4 antibodies (Upstate biotechnology) were used at a concentration of 0.5 µg/ml in phosphate-buffered saline containing 0.1% Tween 20.

### 3. Measurement of lethal activity

Cell death was assayed quantitatively by measuring lactate dehydrogenase (LDH) released in the cell culture medium using Promega CytoTox 96™ kit according to the manufacturer's procedure. The values are expressed as percent LDH activity in the cell culture supernatant with respect to total activity released following Triton-X100 treatment of the cells.

### Example 2 - Electrophysiological characterization of the transfectants

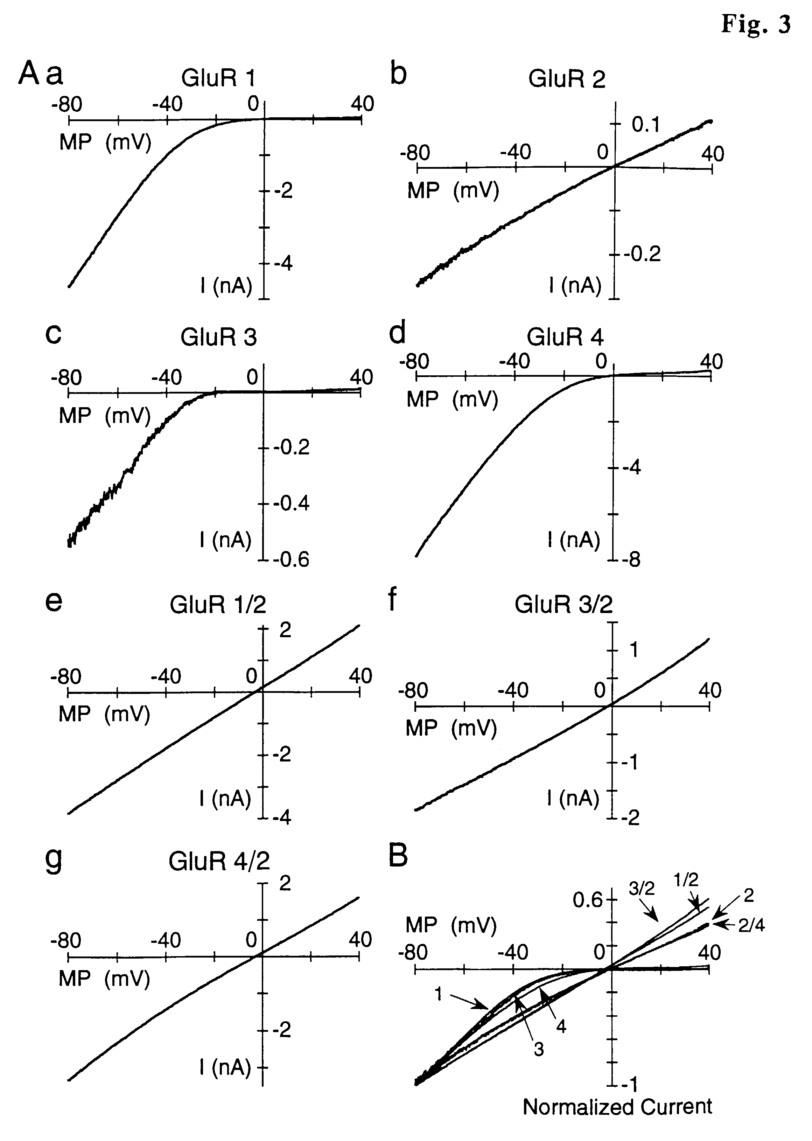

Whole-cell currents were examined to confirm the presence of functional AMPA receptors in the cell lines stably expressing the various AMPA receptor subunits. Cyclothiazide (100 µM) was included in the recording solution to reduce desensitization. Figure 3 shows I-V relationships for 100 µM AMPA-evoked currents obtained from the transfectants stably expressing homomeric and heteromeric AMPA receptor subunits. In the homomeric AMPA receptor expressing cells, GluR1, GluR3 and GluR4 exhibited a marked inward rectification starting around a membrane potential of 0 mV. Marked outward rectifications were observed at potentials more positive than +50 mV (data not shown), exhibiting a doubly rectifying I-V curve. Cells expressing GluR2 subunits, however, behaved in a different fashion in that the I-V curve showed little rectification, giving an almost linear relation. The current amplitudes for GluR2-expressed cells at a membrane potential of -50 mV [-0.16 ± 0.03 nA (mean ± S.E.), n=15] were smaller than those obtained from the other transfectants expressing homomeric AMPA receptor subunits (GluR1: -1.83 ± 0.66 nA, n=10; GluR3: -0.28 ± 0.07 nA, n=15; GluR4: -3.47 ± 0.62 nA, n=19). The transfectants stably co-expressing the different AMPA receptor subunits, such as GluR1/2, GluR3/2 and GluR4/2, displayed I-V relations similar to the homomeric GluR2 receptor. The current amplitudes of the respective heteromeric AMPA receptors (GluR1/2: -2.19 ± 0.28 nA, n=14; GluR3/2: -1.61 ± 0.23 nA, n=14; GluR4/2: -1.18 ± 0.31 nA, n=17) were significantly bigger than those of homomeric GluR2 receptors (see above), even though the transfectant GluR4/2, in which the expression level of GluR4 receptors was very low when tested by western blot (Fig. 1). There were no marked differences in the averaged capacitance values (19.03-21.22 pF) obtained from transfected cell lines.

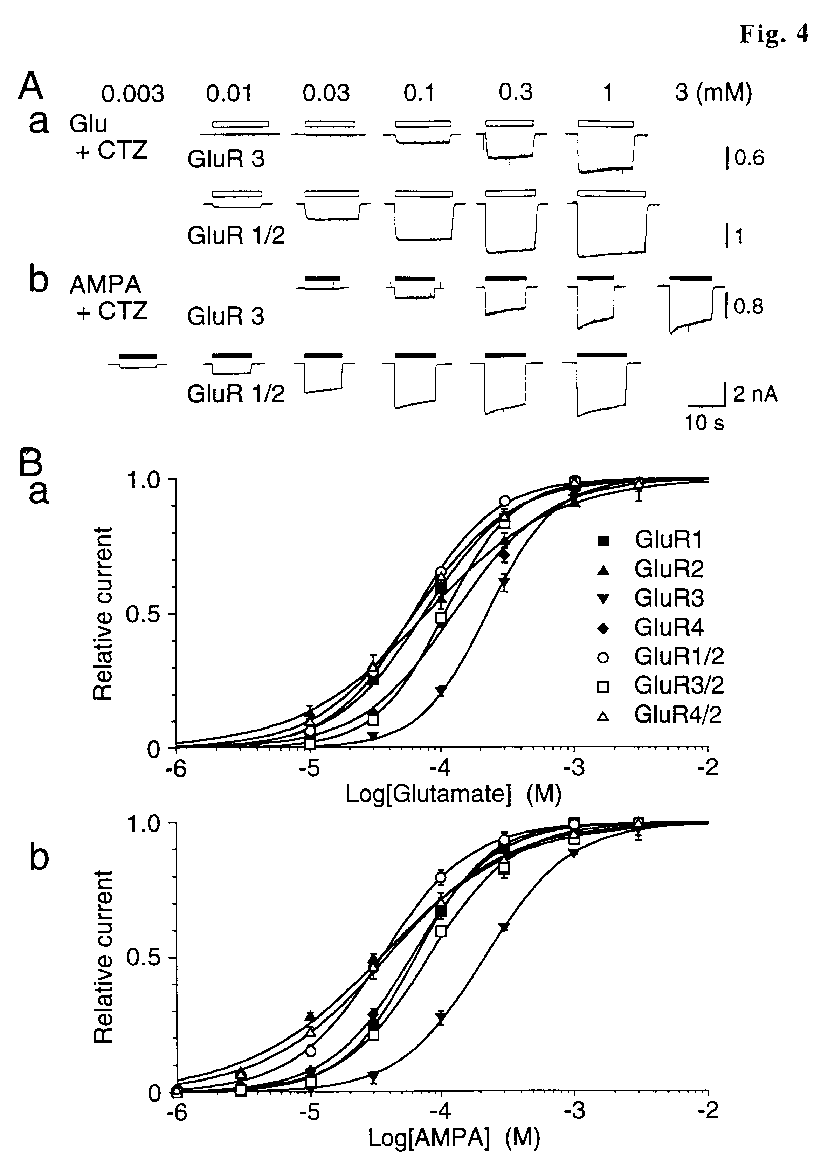

The agonist-evoked current responses in the transfectants stably expressing homomeric and heteromeric AMPA receptors are shown in Fig. 4. Glutamate (10 µM - 1 mM) and AMPA (3 µM - 3 mM) evoked inward currents in a concentration dependent manner (Fig. 4A). In all the cell lines expressing the various AMPA receptor subunits, the EC₅₀ values for AMPA were lower than those for glutamate as summerized in Fig. 4B and Table 1. The clones expressing GluR2 and GluR4 receptors exhibited higher selectivities for AMPA compared to glutamate. Among these recombinant AMPA receptors, GluR3 receptor showed the lowest potency for glutamate- and AMPA-evoked currents.

### Electrophysiology

The studies were carried out using the patch-clamp technique in the whole-cell configuration (Hamill et al., 1981). The intracellular solution, contained in the recording pipette, consisted of 140 mM CsCl, 10 mM HEPES and 10 mM EGTA and the pH adjusted to 7.4 with CsOH. The extracellular solution contained 140 mM NaCI, 5 mM KCl, 2 mM CaCl₂, 10 mM glucose and 10 mM HEPES (adjusted to pH 7.4 with Tris-OH). The agonists were applied with the "Y-tube" method (Wakamori et al., 1993). The agonist-evoked I-V relationships were constructed by ramping the membrane potential from -80 mV to +40 mV for 1.8 seconds. Data obtained in the absence of agonist were subtracted from those acquired during the steady-state component of the response. The concentration-response curves were drawn with a least-squares fitting routine using the following equation: I=Iₘₐₓ x C^{*n*}/(C^{*n*}+EC₅₀^{*n*}), where I denotes the current observed with concentration C, Iₘₐₓ the maximum current, EC₅₀ the half-maximal concentration and *n* the Hill coefficient.

### Example 3 - Binding properties of transfectants stably expressing functional AMPA receptors

### Binding affinities for [³H]AMPA

Scatchard analysis was performed in the concentration of 1 to 100 nM [³H]AMPA using transfectants expressing the GluR1, GluR2, GluR3, and GluR4 alone, and the combinations of GluR2 with GluR1, GluR3, or GluR4 subunits. As shown in Fig. 5, the data points show a straight line, indicating that receptors assembled from homomeric and heteromeric subunits contain a single binding site for [³H]AMPA. The averaged dissociation constant (K_{d}) values calculated from the inverse slopes of the regression lines and the receptor densities (Bₘₐₓ) are summarized in Table 2. The K_{d} values from the transfectants were in the range of 14.5-49.3 nM. In homomeric receptors, GluR2 and GluR3 receptors showed the highest and lowest affinities for [³H]AMPA, respectively. The K_{d} values of GluR1 and GluR4 receptors were intermediate between GluR2 and GluR3. The heteromeric receptors, GluR1/2 and GluR3/2, exhibited higher affinities for [³H]AMPA than those of homomeric GluR1, and GluR3 receptors. In GluR4/2 transfectants, the K_{d} value shifted lower affinity compared to that of the GluR2-expressing cells. The Bₘₐₓ values were in the range of 0.57-7.66 pmol/mg protein.

### Displacement of [³H]AMPA binding by unlabeled glutamate, CNOX, and NS257

In order to examine the pharmacological identity of the stably expressed recombinant receptors, competitive binding studies were performed. Table 3 summarizes the Kᵢ values for glutamate. The calculated Kᵢ values were similar in homomeric and heteromeric AMPA receptors, whereas GluR1 receptor had a tendency to exhibit higher affinity for glutamate. There were no marked differences in Kᵢ values for the competitive antagonists, CNQX and NS257, in the transfectants expressing the various AMPA receptor subunits (Table 4). The ligand displacement potency of CNQX > glutamate > NS257 was similarly recognized in all of these transfectants.

### 1. Membrane preparation and ligand binding

The binding studies were carried out as outlined previously (Keinänen et al., 1990; Fletcher et al., 1995). Briefly, frozen stocks of HEK293 cells transfected with various cDNAs encoding the human AMPA receptor subunits were thawed on ice, suspended in distilled water, homogenized and centrifuged at 50,000 x g for 10 minutes. The pellets were resuspended in 50 mM Tris-HCl (pH 7.5) and repeated the homogenization and centrifugation. The binding assays were performed on cell membrane preparations of 30-50 µg protein per reaction in a tube containing 50 mM Tris-HCl (pH 7.5) with 0.1 M KSCN and the different concentration of [³H]AMPA at a final volume of 0.25 ml. The cell membranes were incubated with the radioligand for 60 minutes on ice, and subsequently collected by rapid filtration through GF/C filters. The nonspecific binding was obtained in the presence of 1 mM glutamate. The filters were washed rapidly with cold assay buffer. The radioactivity retained in the filters was measured by liquid scintillation counting.

### 2. Protein determination

The protein concentration was determined by the protein assay reagent and bovine serum albumin as standard (Bio-Rad).

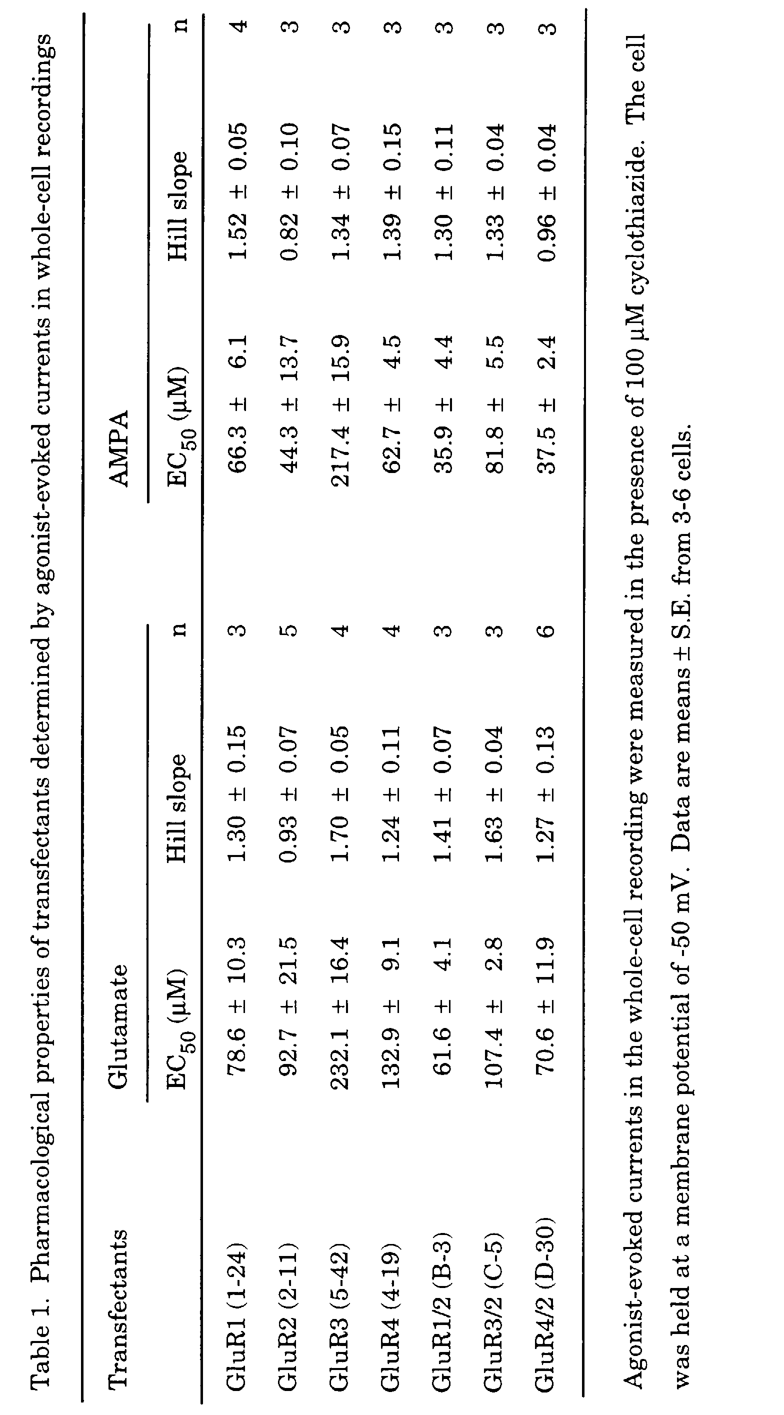

### References

Andersen PH, Tygesen CK, Rasmussen JS, Søegaard-Nielsen L, Hansen A, Hansen K, Kiemer A, and Stidsen CE (1996) Stable expression of homomeric AMPA-selective glutamate receptors in BHK cells. *Eur J Pharmacol* **311:** 95-100.

Burnashev N, Monyer H, Seeburg PH, and Sakmann B (1992a) Divalent ion permeability of AMPA receptor channels is dominated by the edited form of a single subunit. *Neuron* **8:** 189-198.

Burnashev N, Khodorova A, Jonas P, Helm PJ, Wisden W, Monyer H, Seeburg PH, and Sakmann B (1992b) Calcium-permeable AMPA-kainate receptors in fusiform cerebellar glial cells. *Science* **256:** 1566-1570.

Cha J-HJ, Makowiec RL, Penney JB, and Young AB (1992) Multiple states of rat brain (RS)-α-amino-3-hydroxy-5-methylisoxazole-4-propionic acid receptors as revealed by quantitative autoradiography. *Mol Pharmacol* **41:** 832-838.

Condorelli DF, Belluardo N, Mudó G, Dell'Albani P, Jiang X, and Giuffrida-Stella AM (1994) Changes in gene expression of AMPA-selective glutamate receptor subunits induced by status epileptics in rat brain. *Neurochem Int* **25:** 367-376.

Coyle JT and Puttfarcken P (1993) Oxidative stress, glutamate, and neurodegenerative disorders. *Science* **262:** 689-695.

Everall IP, Hudson L, Al-Sarraj S, Honavar M, Lantos P, and Kerwin R (1995) Decreased expression of AMPA receptor messenger RNA and protein in AIDS: a model for HIV-associated neurotoxicity. *Nature Medicine* **1:** 1174-1178.

Fletcher EJ, Nutt SL, Hoo KH, Elliott CE, Korczak B, Mcwhinnie EA, and Kamboj RK (1995) Cloning, expression and pharmacological characterization of a human glutamate receptor: hGluR4. *Receptors and Channels* **3:** 21-31.

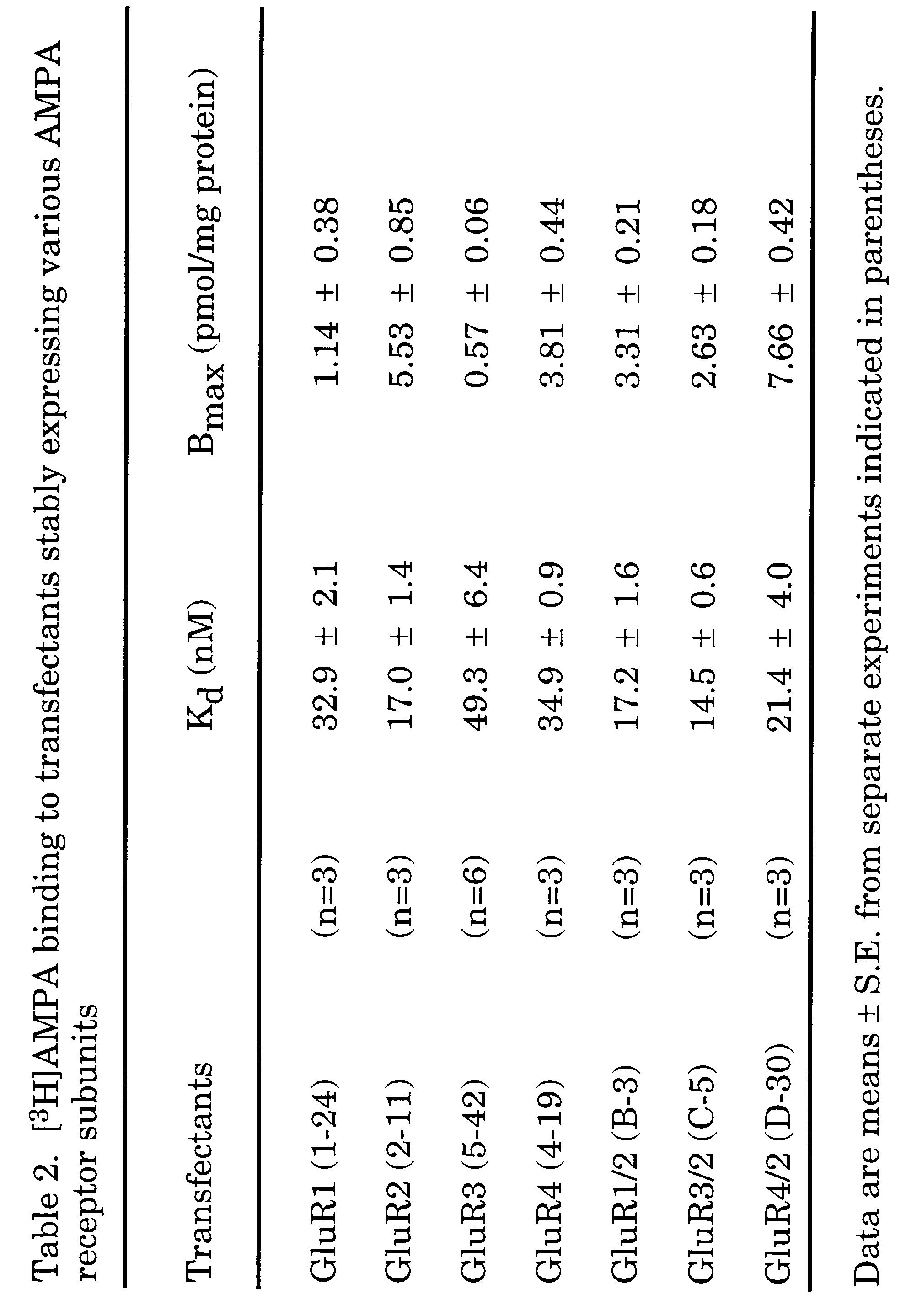

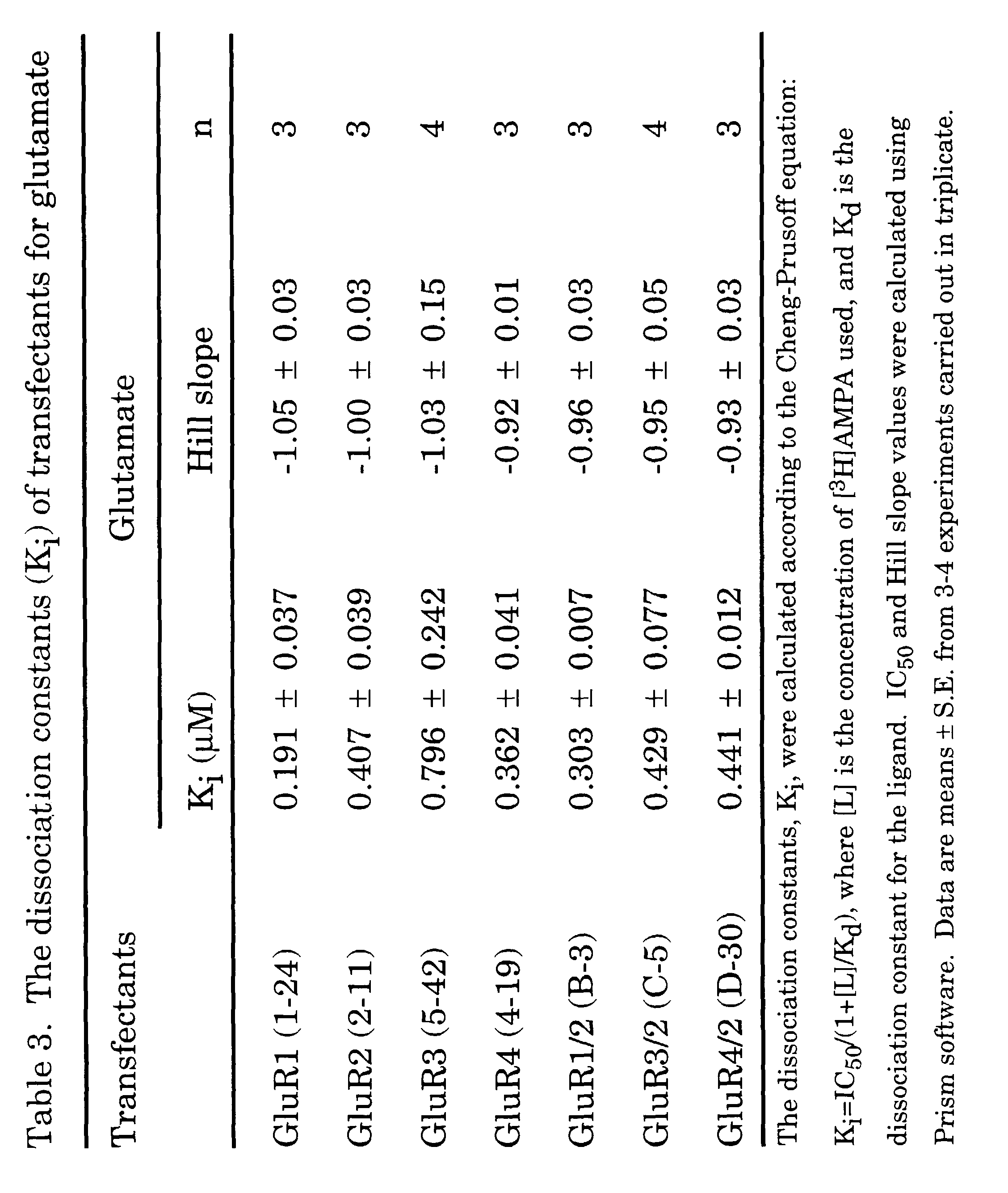

Geiger JRP, Melcher T, Koh D-S, Sakmann B, Seeburg PH, Jonas P, and Monyer H (1995) Relative abundance of subunit mRNAs determines gating and Ca²⁺ permeability of AMPA receptors in principal neurons and interneurons in rat CNS. *Neuron* **15:** 193-204.

Gorter JA, Petrozzino JJ, Aronica EM, Rosenbaum DM, Opitz T, Bennett MVL, Connor JA, and Zukin RS (1997) Global ischemia induces downregulation of GluR2 mRNA and increases AMPA receptor-mediated Ca²⁺ influx in hippocampal CA1 neurons of gerbil. *J Neurosci* **17:** 6179-6188.

Gossen M and Bujard H (1992) Tight control of gene expression in mammalian cells by tetracycline-responsive promoters. *Proc Natl Acad Sci USA* **89:** 5547-5551.

Hamill OP, Marty A, Neher E, Sakmann B, and Sigworth FJ (1981) Improved patch-clamp techniques for high-resolution current recording from cells and cell-free membrane patches. *Pflügers Arch* **391:** 85-100.

Harrison PJ, Barton AJ, Najlerahim A, and Pearson RC (1990) Distribution of a kainate/AMPA receptor mRNA in normal and Alzheimer brain. *NeuroReport* **1:** 149-152.

Hennegriff M, Arai A, Kessler M, Vanderklish P, Mutneja MS, Rogers G, Neve RL, and Lynch G (1997) Stable expression of recombinant AMPA receptor subunits: binding affinities and effects of allosteric modulators. *J Neurochem* **68:** 2424-2434.

Hille, B (1984). Ionic channels of excitable membranes. Sinauer Associates Inc., Sunderland, USA.

Hollmann M, Hartley M, and Heinemann S (1991) Ca²⁺ permeability of KA-AMPA-gated glutamate receptor channels depends on subunit composition. *Science* **252:** 851-853.

Hollmann M and Heinemann S (1994) Cloned glutamate receptors. *Annu Rev Neurosci* **17:** 31-108.

Honoré T and Drejer J (1988) Chaotropic ions affect the conformation of quisqualate receptors in rat cortical membranes. *J Neurochem* **51:** 457-461.

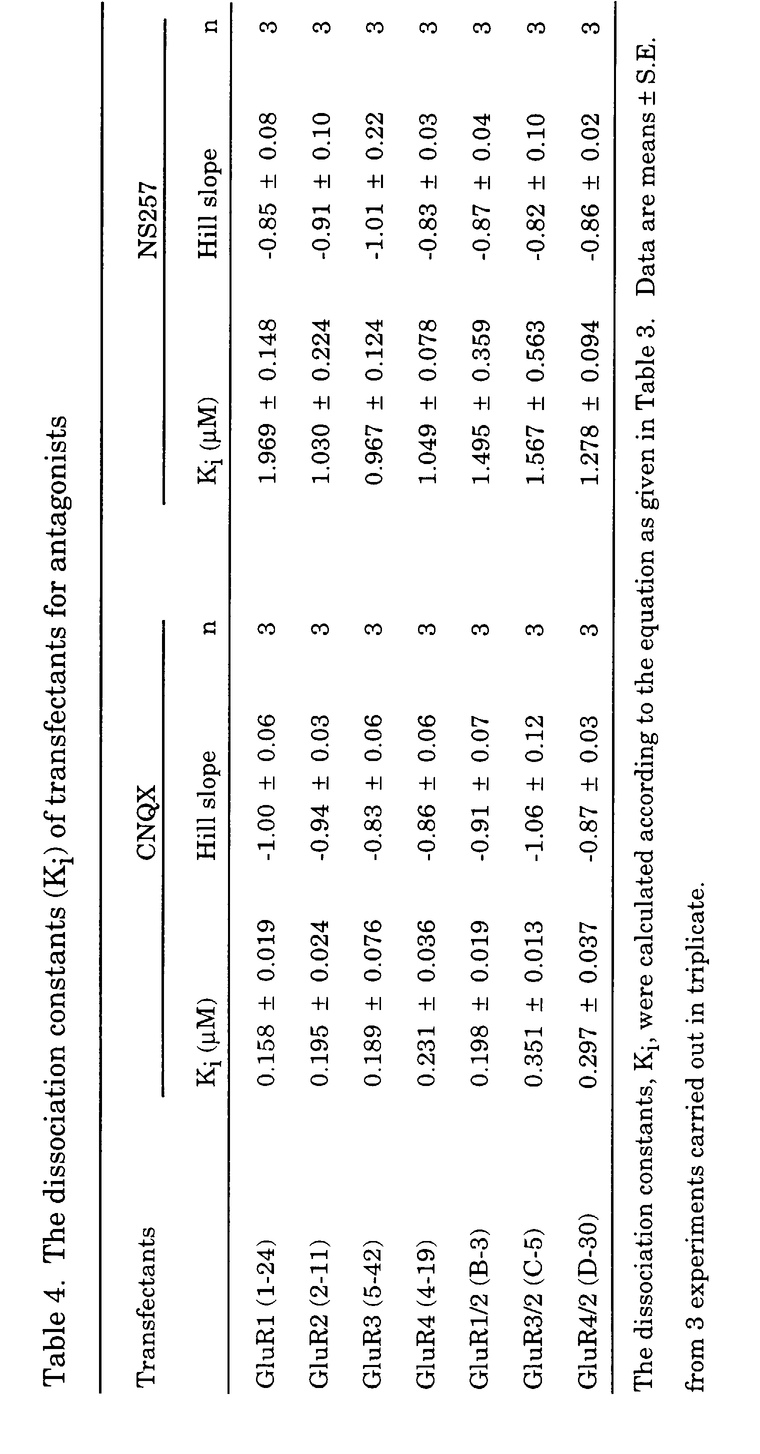

Hume RI, Dingledine R, and Heinemann SF (1991) Identification of a site in glutamate receptor subunits that controls calcium permeability. *Science* **253:** 1028-1031.

Jonas P. AMPA-type glutamate receptors--nonselective cation channels mediating fast excitatory transmission in the CNS (1993). *In:* Siemen D, Hescheler J, eds. Nonselective cation channels: pharmacology, physiology, and biophysics., Birkhäuser Verlag, Basel, Switzerland, **66**, 61-76.

Jonas P and Burnashev N (1995) Molecular mechanisms controlling calcium entry through AMPA-type glutamate receptor channels. *Neuron* **15:** 987-990.

Kamboj SK, Swanson GT, and Cull-Candy SG (1995) Intracellular spermine confers rectification on rat calcium-permeable AMPA and kainate receptors. *J Physiol* **486:** 297-303.

Keinänen K, Wisden W, Sommer B, Werner P, Herb A, Verdoorn TA, Sakmann B, and Seeburg PH (1990) A family of AMPA-selective glutamate receptors. *Science* **249:** 556-560.

Knight MR, Campbell AK, Smith SM, Trewavas AJ. (1991). Recombinant aequorin as a probe for cytosolic free Ca2+ in Escherichia coli. FEBS Lett **282**, 405-408.

Lees, GJ (1996). Therapeutic potential of AMPA receptor ligands in neurological disorders. CNS Drugs, **5:** 51-74.

Mayer ML, Vyklicky L Jr, Westbrook GL (1989). Modulation of excitatory amino acid receptors by group IIB metal cations in cultured mouse hippocampal neurones. J Physiol **415**, 329-350.

Mosbacher J, Schoepfer R, Monyer H, Burnashev N, Seeburg PH, and Ruppersberg JP (1994) A molecular determinant for submillisecond desensitization in glutamate receptors. *Science* **266:** 1059-1062.

Page KJ and Everitt BJ (1995) The distribution of neurons coexpressing immunoreactivity to AMPA-sensitive glutamate receptor subtypes (GluRl-4) and nerve growth factor receptor in the rat basal forebrain. *Eur J Neurosci* **7:** 1022-1033.

Partin KM, Patneau DK, and Mayer ML (1994) Cyclothiazide differentially modulates desensitization of a-amino-3-hydroxy-5-methyl-4-isoxazolepropionic acid receptor splice variants. *Mol Pharmacol* **46:** 129-138.

Pellegrini-Giampietro DE, Zukin RS, Bennett MVL, Cho S, and Pulsinelli WA (1992) Switch in glutamate receptor subunit gene expression in CA1 subfield of hippocampus following global ischemia in rats. *Proc Natl Acad Sci USA* **89:** 10499-10503.

Pellegrini-Giampietro DE, Gorter JA, Bennett MVL, and Zukin RS (1997) The GluR2 (GluR-B) hypothesis: Ca²⁺-permeable AMPA receptors in neurological disorders. *Trends Neurosci* **20:** 464-470.

Pollard H, Heron A, Moreau J, Ben-Ari Y, and Khrestchatisky M (1993) Alterations of the GluR-B AMPA receptor subunit flip/flop expression in kainate-induced epilepsy and ischemia. *Neurosci* **57:** 545-554.

Prasher D, McCann RO, Cormier MJ (1985). Cloning and expression of the cDNA coding for aequorin, a bioluminescent calcium-binding protein. Biochem Biophys Res Commun **126**, 1259-1268.

Remington's Pharmaceutical Sciences. (1990). 18th ed. Mack Publ., Easton.

Sambrook et al. (1989). Molecular Cloning: A Laboratory Manual, 2^{nd} ed., Cold Spring Harbor Laboratory Press, Cold Spring Harbor, New York.

Sheu YA, Kricka LJ, Pritchett DB (1993) Measurement of intracellular calcium using bioluminescent aequorin expressed in human cells. Anal Biochem **209**, 343-347.

Sommer B, Keinänen K, Verdoorn TA, Wisden W, Burnashev N, Herb A, Kohler M, Takagi T, Sakmann B, and Seeburg PH (1990) Flip and flop: a cell-specific functional switch in glutamate-operated channels of the CNS. *Science* **249**: 1580-1585.

Swanson GT, Kamboj SK, and Cull-Candy SG (1997) Single-channel properties of recombinant AMPA receptors depend on RNA editing, splice variation, and subunit *composition. J Neurosci* **17**: 58-69.

Tsien RY (1988). Fluorescence measurement and photochemical manipulation of cytosolic free calcium. Trends Neurosci **11**, 419-424.

Varney MA, Rao SP, Jachec C, Deal C, Hess SD, Daggett LP, Lin F-F, Johnson EC, and Veliçelebi G (1998) Pharmacological characterization of the human ionotropic glutamate receptor subtype GluR3 stably expressed in mammalian cells. *J Pharmacol Exp Ther* **285:** 358-370.

Verdoorn TA, Burnashev N, Monyer H, Seeburg PH, and Sakmann B (1991) Structural determinants of ion flow through recombinant glutamate receptor channels. *Science* **252:** 1715-1718.

Wakamori M, Hidaka H, and Akaike N (1993) Hyperpolarizing muscarinic responses of freshly dissociated rat hippocampal CA1 neurones. *J Physiol* **463:** 585-604.

Washburn MS, Numberger M, Zhang S, and Dingledine R (1997) Differential dependence on GluR2 expression of three characteristic features of AMPA receptors. *J Neurosci* **17:** 9393-9406.

Ying HS, Weishaupt JH, Grabb M, Canzoniero LMT, Sensi SL, Sheline CT, Monyer H, and Choi DW (1997) Sublethal oxygen-glucose deprivation alters hippocampal neuronal AMPA receptor expression and vulnerability to kainate-induced death. *J Neurosci* **17:** 9536-9544.

Yoneda Y and Ogita K (1991) Neurochemical aspects of the N-methyl-D-aspartate receptor complex. *Neurosci Res* **10:** 1-33.

## Claims

1. Cell line stably expressing a recombinant heteromeric AMPA glutamate receptor on its cell surface.

2. Cell line according to claim 1, wherein said receptor comprises the glutamate receptor 1 subunit (GluR1).

3. Cell line according to claim 1 or 2, wherein said receptor comprises the glutamate receptor 2 subunit (GluR2).

4. Cell line according to any one of claims 1 to 3, wherein said receptor comprises the glutamate receptor 3 subunit (GluR3).

5. Cell line according to any one of claims 1 to 4, wherein said receptor comprises the glutamate receptor 4 subunit (GluR4).

6. Cell line according to any one of claims 1 to 5, wherein said receptor comprises of the glutamate receptor 1 (GluR1) and the glutamate receptor 2 subunit (GluR2).

7. Cell line according to any one of claims 1 to 6, wherein said receptor comprises of the glutamate receptor 1 (GluR1) and the glutamate receptor 3 subunit (GluR3).

8. Cell line according to any one of claims 1 to 7, wherein said receptor comprises of the glutamate receptor 1 (GluR1) and the glutamate receptor 4 subunit (GluR4).

9. Cell line according to any one of claims 1 to 8, wherein said receptor comprises of the glutamate receptor 2 (GluR2) and the glutamate receptor 3 subunit (GluR3).

10. Cell line according to any one of claims 1 to 9, wherein said receptor comprises of the glutamate receptor 2 (GluR2) and the glutamate receptor 4 subunit (GluR4).

11. Cell line according to any one of claims 1 to 10, wherein said receptor comprises of the glutamate receptor 3 (GluR3) and the glutamate receptor 4 subunit (GluR4).

12. Cell line according to any one of claims 1 to 11, wherein said receptor comprises five glutamate receptor subunits (GluR) according to any one of claims 3 to 6.

13. Cell line according to any one of claims 1 to 12, wherein said cell line expresses a recombinant human AMPA glutamate receptor.

14. Cell line stably expressing a recombinant human homomeric AMPA glutamate receptor wherein said receptor comprises at least two glutamate receptor 1 subunits (GluR1).

15. Cell line stably expressing a recombinant human homomeric AMPA glutamate receptor wherein said receptor comprises at least two glutamate receptor 2 subunits (GluR2).

16. Cell line stably expressing a human homomeric AMPA glutamate receptor wherein said receptor comprises at least two glutamate receptor 3 subunits (GluR3).

17. Cell line stably expressing a human homomeric AMPA glutamate receptor wherein said receptor comprises at least two glutamate receptor 4 subunits (GluR4).

18. Cell line according to any one of claims 14 to 17, wherein said receptor comprises five glutamate receptor subunits (GluR).

19. Cell line according to any one of claims 1 to 18, wherein said receptor comprises the flip splice variant of the subunit.

20. Cell line according to any one of claims 1 to 19, wherein said receptor comprises the flop splice variant of the subunit.

21. Cell line stably expressing a recombinant human homomeric AMPA glutamate receptor wherein said receptor comprises at least two flop splice variants of the glutamate receptor 3 subunit (GluR3).

22. Cell line stably expressing a recombinant human homomeric AMPA glutamate receptor wherein said receptor comprises at least two flop splice variants of the glutamate receptor 4 subunit (GluR4).

23. Cell line according to any one of claims 1 to 22 which is derived from the HEK 293 cell line.

24. HEK 293 cell line stably expressing a recombinant human homomeric AMPA glutamate receptor on its cell surface, wherein said receptor consists of the flip splice variant of the glutamate receptor 1 (GluR1).

25. HEK 293 cell line stably expressing a recombinant human homomeric AMPA glutamate receptor on its cell surface, wherein said receptor consists of the flip splice variant of the glutamate receptor 2 (GluR2).

26. HEK 293 cell line stably expressing a recombinant human homomeric AMPA glutamate receptor on its cell surface, wherein said receptor consists of the flip splice variant of the glutamate receptor 3 (GluR3).

27. HEK 293 cell line stably expressing a recombinant human homomeric AMPA glutamate receptor on its cell surface, wherein said receptor consists of the flip splice variant of the glutamate receptor 4 (GluR4).

28. HEK 293 cell line stably expressing a recombinant human heteromeric AMPA glutamate receptor on its cell surface, wherein said receptor consists of the flip splice variants of the glutamate receptor 1 (GluR1) and the glutamate receptor 2 subunits (GluR2).

29. HEK 293 cell line stably expressing a recombinant human heteromeric AMPA glutamate receptor on its cell surface, wherein said receptor consists of the flip splice variants of the glutamate receptor 2 (GluR2) and the glutamate receptor 3 subunits (GluR3).

30. HEK 293 cell line stably expressing a recombinant human heteromeric AMPA glutamate receptor on its cell surface, wherein said receptor consists of the flip splice variants of the glutamate receptor 2 (GluR2) and the glutamate receptor 4 subunits (GluR4).

31. Method for preparing a cell line according to any one of claims 1 to 30 comprising the following steps:
a) construction of an expression vector which comprises DNA specific for a glutamate receptor subunit
b) transfection of a suitable cell line with said expression vector
c) screening said cell line for stable transfection employing a suitable selection agent.

32. Method according to claim 31, wherein said cell line is co-transfected with two or more of said expression vectors each of which is comprising DNA specific for a different glutamate receptor subunit.

33. Use of a cell line according to any one of claims 1 to 30 for the identification of glutamate receptor agonists.

34. Use of a cell line according to any one of claims 1 to 30 for the identification of glutamate receptor antagonists or modulators.

35. Use of a cell line according to any one of claims 1 to 30 in a high throughput screening (HTS) format.

36. Use of a cell line according to any one of claims 1 to 30 in an electrophysiological assay with an agonist, antagonist or modulator of a glutamate receptor wherein membrane currents are measured.

37. Use of a cell line according to any one of claims 1 to 30 in a receptor-binding assay.

38. Use of a cell line according to any one of claims 1 to 30 in a fluorometric assay wherein the intracellular quantity of a divalent ion is measured.

39. Method for the identification of an agonist, antagonist or modulator of a glutamate receptor wherein a cell line according to any one of claims 1 to 30 is incubated with a test substance.

40. Method according to claim 39, further characterized by the following steps:
a) a membrane current is measured
b) said membrane current is compared with the membrane current obtained for said cell line after incubation with a known control or in the absence of the test substance.

41. Method according to any one of claims 39 or 40, wherein said method is characterized by the following steps:
a) a cell line according to any one of claims 1 to 30 is incubated with a known agonist which is coupled to a reporter
b) said cell line is incubated with a test substance
c) the displacement of the agonist coupled to the reporter by the test substance is measured.

42. Method according to any one of claims 39 to 41, wherein said method is characterized by the following steps:
a) a cell line according to any one of claims 1 to 30 is incubated with a test substance
b) the intracellular quantity of a divalent ion is measured
c) said quantity of the intracellular divalent cation is compared with the quantity of the intracellular divalent cation measured for said cell line after incubation with a known control or in the absence of the test substance.

43. Method according to any one of claims 39 to 42, wherein said method is a high throughput screening assay (HTS).

44. Pharmaceutical composition comprising an agonist, antagonist or modulator of a glutamate receptor identifiable with a method according to any one of claims 39 to 43 and a pharmaceutically acceptable carrier.

45. Use of an agonist, antagonist or modulator of a glutamate receptor identifiable with a method according to any one of claims 39 to 43 in the manufacture of a medicament for the treatment of neurological disorders.
